# EUROPEAN PATENT APPLICATION

(11) **EP 2 696 199 A1**
(43) Date of publication of application: **12.02.2014**
(21) Application number: 13166160.5
(22) Date of filing: 02.05.2013
(51) Int. Cl.: G01N 33/487

(54) **Electrochemical test strip reading apparatus and method**

(30) Priority: 10.08.2012 TW 101129029
(71) Applicant: Actherm Inc., 30078 Hsinchu City (TW)
(72) Inventor: Chen, Min-Ying, 303 Hsinchu County (TW); Hsieh, Miin-Jsia, 111 Taipei City (TW)
(74) Representative: Hintermeyer, Julia

(57) **Abstract**

The invention discloses an electrochemical test strip reading apparatus and method. The electrochemical test strip reading apparatus (100) comprises a housing (101), a monitor (102), a slot (103) for an electrochemical test strip (20) and a code card (10), a current detecting and converting circuit (104), and a microprocessor (105). The code card (10) not only provides complete identification information for the electrochemical test strip (20), but also is used as a memory card and a calibration card.

## Description

### BACKGROUND OF THE INVENTION

### 1. Technical Field

The present invention is related to a biological detecting strip reading apparatus and method, and more particularly related to an electrochemical strip reading apparatus and method.

### 2. Description of Related Art

According to the progress of the civilization, the incidence and prevalence of the diabetes increase year after year and the morbidity age decrease year after year. In recent years, diabetes is top five in ten leading causes of death in Taiwan area. As a matter of fact, the diabetes is in close relation with the top ten causes of death including heart disease, high pressure and stroke. Accordingly, the medical faults keep looking for clinical trials related to diabetes to find out a proper cure and also promote the well control of blood sugar to reduce the generation of the complication of diabetes. Therefore, the blood sugar detection is not only used in determining the blood sugar value in plasma in laboratory, but also developed to be a small blood sugar detector for users to detect at home. The user can detect the blood sugar himself to get the result quickly so as to decide to change the treatment, shorten the patient's hospitalization time or avoid unnecessary clinic hours. It is an importance milestone in diabetes treatment history.

The blood sugar detective system can be an optical detector or an electrochemical detector. The optical detector is to implement glucose oxidase (GOD) to react with glucose in blood to generate hydrogen peroxide. The hydrogen peroxide reacts with Horseradish Peroxidase (HRPO) to generate a color and the color can be detected by a reflective photometer or an absorption photometer. However, the optical detector is required to eliminate red blood cells to avoid any reaction caused by red blood cells and it is easy to cause the operators to be polluted and result in a huge measurement error if the detective operation is not accurate. In order to increase the accuracy, the electrochemical detector, which is not required to eliminate glucose dehydrogenase (GDH), is used in recent years and the current generated by the reaction is converted to be a blood sugar value shown on the monitor. The method not only avoids the error generated by the optical comparing process but also simplifies the operative steps for the patients, and reduces the detective time and the usage amount of the blood. Besides, the price of the electrochemical detector is cheaper, so the electrochemical detector is more and more popular.

In addition, according to the studies of related literatures, if the user operates correctly, the quality of the small blood sugar detector is reliable and the proper detective record of the blood sugar can provide a significant reference for making a proper treatment in clinical. In order to make sure the detective result is correct, the detector manufacturers develop a blood sugar detector with an additional adjusted chip. The adjusted chip can store some identifying information, such as types, serial numbers or expiration date, of the strips. During detecting, the adjusted chip corresponding to the type and the serial number of the strip is inserted into the detector, the identifying information of the adjusted chip is read by the detector and shown on the monitor, thus the user can determine if the detective strip is the same as the type and serial number of the strip shown on the monitor or the strip is expired. Because the adjusted chip may also include the corrected information of the detective value belonged to the strip, it is required to use the detective strip with the same serial number as the adjusted chip to make sure the detective result is accurate.

However, the aging problem exists in all detectors. When the detector is getting old, the detective result may be affected and lost its accuracy. Therefore, how to determine the detective result is correct, how to adjust the detective value efficiently, and how to solve the aging problem of the detectors are all the goals that the developer would like to improve.

### SUMMARY OF THE INVENTION

In order to solve the problems described in prior art, an electrochemical strip reading apparatus and method are disclosed in the present invention to read the identifying information of an individual code card and the detecting signal of an electrochemical strip. The apparatus provided in the present invention includes a housing, a monitor, a slot, a current detecting and converting circuit and a microprocessor. The monitor is disposed on the housing to display the detecting result. The slot includes a plurality of pins and is disposed on the housing and is provided for insertion of the code card and the electrochemical strip. The current detecting and converting circuit is disposed within the housing to read a standard resistor signal of the standard resistor of the code card and a fluid reacting signal of a fluid reacting area of the electrochemical strip via the slot. The microprocessor is disposed within the housing and comprises a database module and a calculating module. The database module stores a plurality of identifying information including the one stored in the inserted code card and the fluid reacting signals. The calculating module reads the identifying information of the electrochemical strip in the database module and calculates a detecting result by comparing the fluid reacting signal and the identifying information.

The code card is connected to the slot of the electrochemical strip reading apparatus for recognition via the metal pin area which comprises a plurality of metal pins. When the electrochemical strip reading apparatus recognizes the code card, the electrochemical strip reading apparatus reads the identifying information including the type, the serial number, the expiring date, the related calculation formula, and the reagent correction data of the electrochemical strip. Meanwhile, the electrochemical strip reading apparatus reads the standard resistor reference value stored in the memory chip, reads the standard resistor signal of the standard resistor, and obtains a calibration deviation value by comparing the standard resistor reference value and the standard resistor signal. The electrochemical strip reading apparatus automatically uses the calibration deviation value as a compensation value to correct the detection error occurred in reading the fluid reacting signal of the electrochemical strip, and thus a correct data of blood sugar is obtained.

Therefore, the main object of the present invention is to provide an electrochemical strip reading apparatus and method thereof. When the electrochemical strip reading apparatus recognizes the code card, it can read the identifying information of the code card and store the identifying information in the electrochemical strip reading apparatus. The method disclosed in the present invention is different from the conventional one by simultaneously inserting a code card and a strip to complete the detection. Hence, the problem that the information cannot be obtained when the code card is unavailable can be solved.

The second object of the present invention is to provide an electrochemical strip reading apparatus and method thereof. When the electrochemical strip reading apparatus recognizes the code card, it reads the identifying information of the code card and stores the identifying information in the electrochemical strip reading apparatus. Further, the code card is designed to have a standard resistor in the code card and store a standard resistor reference value in the memory chip of the code card. By reading the standard resistor signal of the standard resistor and comparing the standard resistor signal with the standard resistor reference value, a calibration deviation value is obtained and used to correct the detecting error. Therefore, the detecting error caused by the aging problem or the connecting problem of the equipment can be improved so as to increase the accuracy of the detecting result.

Another object of the present invention is to provide a reading method for the electrochemical strip to detect the detecting signal released from the chemical reaction of the sample on the electrochemical strip. The detecting signal is processed by conversion, comparison, calculation and correction to display the detecting result, and thus the detecting procedure is completely accomplished.

One another object of the present invention is to provide a reading method for the code card to read the identifying information and the standard resistor reference value of the code card and read the standard resistor signal of the standard resistor on the code card. Next, the standard resistor reference value is compared with the stand resistor signal and the comparing result is utilized to correct the calibrating deviation of the electrochemical strip reading apparatus. Moreover, the detecting results recorded in the electrochemical strip reading apparatus are restored in the code card. The code card can store many data, including the detecting results of the same batch of electrochemical strips, the identifying information of various code cards in different periods, and the detecting results of the different batches of electrochemical strips. Hence, the code card can be used as a case history storing card for long time recording detecting results, highly contributing to the long-distance medical consultant and tracking.

### BRIEF DESCRIPTION OF THE DRAWINGS

The foregoing aspects and many of the attendant advantages of this invention will become more readily appreciated as the same becomes better understood by reference to the following detailed description, when taken in conjunction with the accompanying drawings, wherein:
FIG. 1A is a structural view illustrating an electrochemical strip reading apparatus and a code card used for the apparatus in a first and second embodiment of the present invention;
FIG. 1B is a structural view illustrating a connection relationship between the pins of the slot and the metal pins of the code card in the electrochemical strip reading apparatus in the first and second embodiment of the present invention;
FIG. 2A is a structural view illustrating an electrochemical strip reading apparatus and an electrochemical strip used for the apparatus in the first and second embodiment of the present invention;
FIG. 2B is a structural view illustrating a connection relationship between the pins of the slot and the electrodes of the electrochemical strip in the electrochemical strip reading apparatus in a first and second embodiment of the present invention;
FIG. 3A is a flow chart illustrating a code card reading method in one embodiment of the present invention; and
FIG. 3B is a flow chart illustrating an electrochemical strip reading method in one embodiment of the present invention.

### DETAIL DESCRIPTION OF THE PREFERRED EMBODIMENT

Some particular embodiments of the invention will be described in detail for purpose of illustration, and one of ordinary skill in the art can easily understand the advantages and efficacy of the present invention through the disclosure of the specification. It is to be understood that alternative embodiments may be possible for the implement and application of the present invention while numerous variations will be possible to the details disclosed in the specification on the strength of diverse concepts and applications without going outside the scope of the invention as disclosed in the claims.

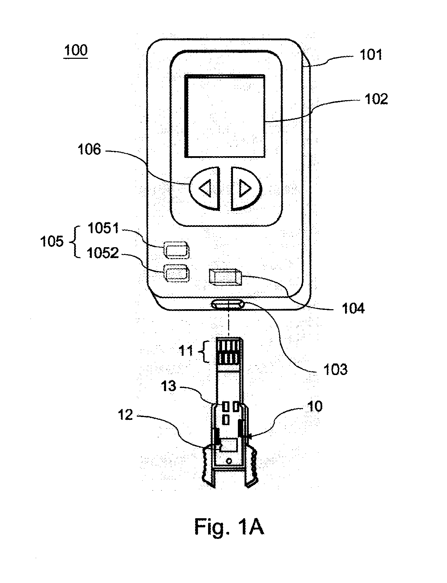

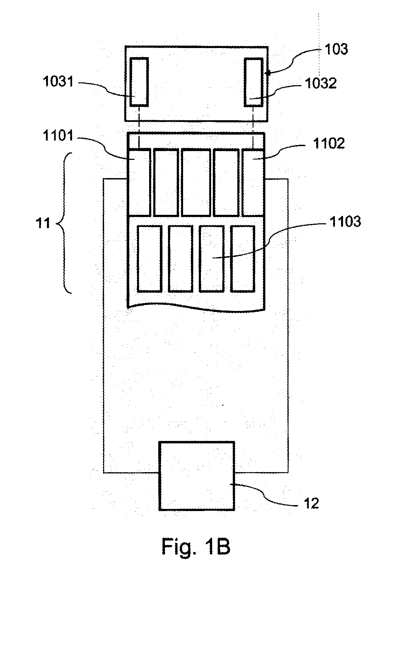

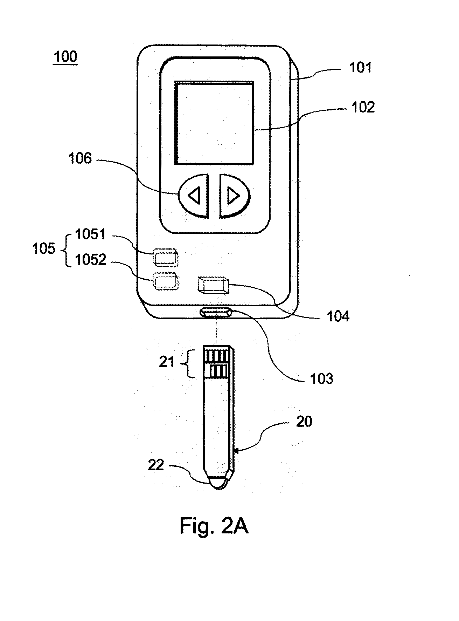

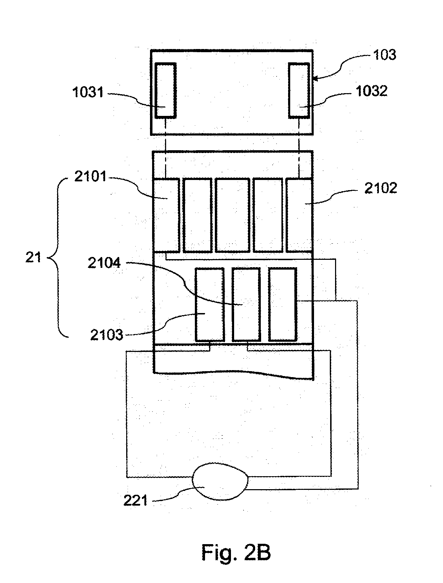

Please refer to FIGs. 1A, 1B, 2A and 2B, which illustrate a first embodiment of the present invention. The electrochemical strip reading apparatus 100 is configured to read a code card 10 and an electrochemical strip 20. Specifically, the electrochemical strip reading apparatus 100 is to read the identifying information stored in the code card 10 and the fluid reacting signal transmitted from the electrochemical strip 20. The electrochemical strip reading apparatus 100 includes a code card 10, a housing 101, a monitor 102, a slot 103, a current detecting and converting circuit 104, a microprocessor 105 and an operating button 106.

The monitor 102 is disposed on the housing 101 and configured to display the detecting result. The slot 103 is disposed on the housing 101 for insertion of the code card 10 and the electrochemical strip 20. The slot 103 includes a plurality of pins 1031, 1032 for connecting with the metal pin area 11 of the code card 10 and the electrical area 21 of the electrochemical strip 20. The current detecting and converting circuit 104 is disposed within the housing 101 to read a fluid reacting signal released from the fluid reacting area 22 of the electrochemical strip 20. The microprocessor 105 is disposed within the housing 101 and includes a database module 1051 and a calculating module 1052. The database module 1051 stores the standard resistor reference value, the standard resistor signal and the fluid reacting signal, and a plurality of identifying information including the identifying information of the currently utilized electrochemical strip and other identifying information of other electrochemical strips. Each of the identifying information includes the type, the serial number, the calculation formula, the expired date, and the reagent correction data of the electrochemical strip 20 and so on. The calculating module 1052 compares the type and serial number of the corresponding identifying information of the electrochemical strip in the database module 1051 with the type and serial number of the electrochemical strip 20 and reads the corresponding calculation formula and reagent correction data. After calculating, the detecting result representing the fluid reacting signal is obtained and displayed on the monitor 102. In addition, the electrochemical strip reading apparatus 100 provides the operation buttons 106 for selection of steps during detection.

The code card 10 includes a metal pin area 11 and a memory chip 12. The memory chip 12 includes the corresponding identifying information for the electrochemical strip 20, such as the type, serial number, expired date, calculation formula, reagent correction data of the electrochemical strip 20 and so on. The metal pins 1101, 1102 formed in the metal pin area 11 of the code card 10 are used for connecting with the pins 1031, 1032 of the slot 103 of the electrochemical strip reading apparatus 100, thus providing a way of identification and data transmission. As the metal pin 1101 is connected with the pin 1031 and the metal pin 1102 is connected with the pin 1032, the electrochemical strip reading apparatus 100 can recognize the code card 10.

When the electrochemical strip reading apparatus 100 recognizes the code card 10, the electrochemical strip reading apparatus 100 is activated (the activating method can be automatic or manual and the automatic activating method is preferred to be used in the present embodiment). Subsequently, the electrochemical strip reading apparatus 100 reads the identifying information stored in the memory chip 12 of the code card 10 and stores the identifying information in the database module 1051 of the microprocessor 105 for comparing with the fluid reacting signal transmitted from the electrochemical strip 20 to provide a detecting result calculated by the calculating module 1052.

Moreover, the memory chip 12 disposed on the code card 10 can automatically transmit the identifying information to the database module 1051 of the microprocessor 105 as well as store the identifying information therein. Also, the memory chip 12 of the code card 10 can transmit the detecting results stored in the database module 1051 to the memory chip 12 of the code card 10 and store the detecting results therein. Therefore, the memory chip 12 in the present invention can transmit and store the identifying information in the database module 1051 of the microprocessor 105, and restore the detecting results stored in the database module 1051 to the memory chip 12 of the code card 10. That is, a bi-directional transmitting of identifying information and detecting result is implemented in the present invention. Further, the memory chip 12 of the code card 10 can access the plurality of detecting results stored in the database module 1051 and serves as a bi-directional transmitting interface between the code card 10 and the electrochemical strip reading apparatus 100. Hence, output of the detecting results and usage information by the code card 10 served as an interface is carried out by a decoder in remote interpretation.

The electrochemical strip 20 in the present invention includes an electrical area 21 and a fluid reacting area 22, and forms at least two electrodes 2101, 2102 in the electrical area 21 for a respective connection with the pins 1031, 1032 of the slot 103. Besides, the electrode 2101 is connected to the pin 1031 as well as the electrode 2102 is connected to the pin 1032, and the respective connection enables the electrochemical strip reading apparatus 100 to recognize the electrochemical strip 20. The electrical area 21 includes another electrodes 2103, 2104 for connecting with the blood sample 221 of the fluid reacting area 22 to detect the fluid reacting signal generated by chemical reaction.

After verification, if the electrochemical strip reading apparatus 100 recognizes it a correct electrochemical strip 20, the electrochemical strip reading apparatus 100 is activated. Then, the fluid reacting signal generated from the blood sample 221 on the fluid reacting area 22 of the electrochemical strip 20 can be detected. Because the electrodes 2103, 2104 are connected to the blood sample 221 on the fluid reacting area 22, the fluid reacting signal generated by the chemical reaction occurred in the blood sample 221 is transmitted to the electrochemical strip reading apparatus 100 by the respective connection between the electrical area 21 of the electrochemical strip 20 and the pins of the slot 103 of the electrochemical strip reading apparatus 100. Thereafter, the current detecting and converting circuit 104 reads the fluid reacting signal generated by the fluid reacting area 22 of the electrochemical strip 20 and compares the fluid reacting signal with the identifying information stored in the database module 1051. Also, the calculating module 1052 calculates the detecting result based on the calculation formula and the reagent correction data corresponding to the electrochemical strip 20 and displays the calculated result on the monitor 102.

Conversely, if the electrochemical strip reading apparatus 100 recognizes it a wrong electrochemical strip 20 after verification, the electrochemical strip reading apparatus 100 cannot be activated. At the same time, the electrochemical strip 20 is removed and the electrochemical strip reading apparatus 100 exits the operation mode so as to guarantee the correct electrochemical strip 20 being used.

Due to the identifying information originally stored in the memory strip 12 of the code card 10 can be automatically transmitted and stored in the electrochemical strip reading apparatus 100, thus whether the electrochemical strip 20 is correct can be determined after verification. Comparing with the conventional equipment required to conduct the detection under the existence of a calibration card, the electrochemical strip reading apparatus 100 in the present invention records all the read identifying information, thereby providing a way of more convenient detection. Accordingly, the problem occurred in the conventional equipment such as losing the calibration card and resulting in loss of the identifying information of the electrochemical strip 20 can be solved. In addition, the code card 10 of the present invention can be read by the decoder to execute the medical service at a long distance, thus the long-distance medical consultant and tracking is achieved via remote connection, highly contributing to home care and long-distance medical care.

Please still refer to FIGs. 1A, 1B, 2A and 2B, in addition to the first embodiment, in which the identifying information loaded in the code card 10 is automatically stored into the electrochemical strip reading apparatus 100 to provide information required by detecting the electrochemical strip 20, the present invention further illustrates the second embodiment to provide a way of precise detection. Hence, in the second embodiment of the present invention, the code card 10 is designed to further comprise a standard resistor 13 with a standard resistor signal detected by the electrochemical strip reading apparatus 100. Further, the stand resistor reference value stored in the memory chip 12 is designed to compare with the standard resistor signal for assisting the electrochemical strip reading apparatus 100 in executing the correction process, thereby preventing from the detecting error generated by aging of the equipment.

As shown in FIGs. 1A and 1B, when the metal pins 1101, 1102 of the code card 10 is connected with the pins 1031, 1032 of the slot 103, the electrochemical strip reading apparatus 100 recognizes the code card 10. Then, the electrochemical strip reading apparatus 100 not only reads the identifying information including the type, serial number, expired date, calculation formula and reagent correction data stored in the memory chip 12 of the code card 10 but also reads the standard resistor reference values stored in the memory chip 12. The identifying information and the standard resistor reference values are transmitted and stored into the database module 1051 of the microprocessor 105. In addition, the standard resistor 13 disposed on the code card 10 is connected to the metal pin 1103 of the metal pins area 11. The electrochemical strip reading apparatus 100 reads the signal generated by the standard resistor 13 on the code card 10 via the pins 1031, 1032 of the slot 103. Subsequently, the signal is transmitted to the current detecting and converting circuit 104 and then calculated by the calculating module 1052 of the microprocessor 105 to obtain the standard resistor signal. The stand resistor signal is stored into the database module 1051 in order to be utilized in comparing with the stand resistor reference value within the database module 1051 so as to obtain a calibration deviation value. Thereafter, the calibration deviation value is restored into the database module 1051 of the database module 105 and served as the compensation value for correcting the detecting error generated from the electrochemical strip reading apparatus 100 detecting the electrochemical strip 20. That is, the detecting error is automatically calibrated by the compensation value.

Therefore, please refer to FIGs. 1A and 1B, the electrochemical strip reading apparatus 100 in the second embodiment of the present invention not only reads the identifying information of the electrochemical strip 20 stored in the memory chip 12 of the code card 10 but also reads the standard resistor reference value of the standard resistor 13 stored in the memory chip 12, and stores the identifying information and the standard resistor reference value into the database module 1051 for comparing with the fluid reacting signal of the electrochemical strip 20 to obtain a detecting result. On the other hand, the electrochemical strip reading apparatus 100 obtains the standard resistor signal by connecting with the standard resistor 13 of the code card 10. The stand resistor signal is compared with the standard resistor reference value to generate a calibration deviation value. The calibration deviation value is restored into the database module 1051 of the electrochemical strip reading apparatus 100 to indicate that there is a detecting error existing in the electrochemical strip reading apparatus 100. Also, the calibration deviation value will be the compensation value for the following detection of the electrochemical strip 20. Hence, the electrochemical strip reading apparatus 100 will compensate the standard resistor signal automatically if the calibration deviation value is within a tolerance range and the detecting result obtained by the correction procedure is more accurate and valuable for medical reference.

Please refer to FIGs. 2A and 2B, according to the correction procedure of the electrochemical strip reading apparatus 100, when the electrodes 2101, 2102 in the electrical area 21 of the electrochemical strip 20 are connected with the pins 1031, 1032 of the slot 103, the electrochemical strip reading apparatus 100 will determine if it is the electrochemical strip by the connection relationship between the electrodes and the pins. Then, checking the identifying information of the electrochemical strip 20 is executed to determine if it is the correct electrochemical strip 20. If it is a correct electrochemical strip 20, the electrochemical strip reading apparatus 100 is activated to enter the operation mode. Thereafter, the sample is injected into the fluid reacting area 22 to generate a fluid reacting signal by chemical reaction, and the fluid reacting signal is read by the current detecting and converting circuit 104. Subsequently, the calibration deviation value is compensated to the fluid reacting signal by the automatic compensating error procedure of the electrochemical strip reading apparatus 100, and the compensated value is calculated by the calculating module 1052 to obtain the detecting result.

For example, before a blood sugar test, the electrochemical strip reading apparatus 100 executes the correction procedure first via the code card 10. If the value of the standard resistor signal detected by the electrochemical strip reading apparatus 100 is 150, which is equal to the standard resistor reference value 150 stored in the database module 1051, the compensation value obtained by calculating the difference between the standard resistor signal and the standard resistor reference value is 0. Accordingly, the detecting result read by the electrochemical strip reading apparatus 100 is correct. However, when the machine is getting older and the connection relationship between the machine and the inserting strips would be malfunctioned, thus the value of the standard resistor signal may be reduced to 145. By comparing with the stand resistor reference value 150 stored in the database module 1051 and calculating the difference, the compensation value 5 is obtained and stored into the database module 1051 of the electrochemical strip reading apparatus 100. Accordingly, an error warning existing in the electrochemical strip reading apparatus 100 is provided and the detecting result of the electrochemical strip 20 is compensated by the compensation value. Furthermore, if the calibration deviation value is within a tolerable range, the detecting result of the electrochemical strip 20 is automatically compensated.

The present invention not only automatically stores the identifying information, such as the type, serial number, expired date and calculation formula corresponding to the electrochemical strip 20, into the electrochemical strip reading apparatus 100 for determining if the electrochemical strip 20 is correct or not. Also, it provides an automatic compensation procedure to correct the detecting error for the electrochemical strip reading apparatus 100. Hence, the present invention provides a way of more convenient detection and high accuracy in the detecting result.

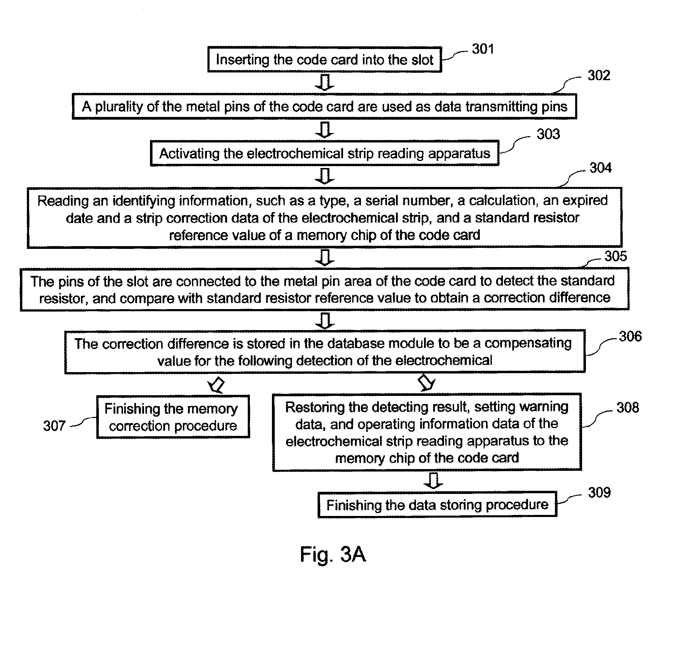

The present invention further provides an electrochemical strip reading method using the electrochemical strip reading apparatus 100 described above. Next, please base on the structural views shown in FIGs 1A, 1B, 2A and 2B and refer to the flow chart shown in FIGs 3A and 3A.

Please refer to FIG. 3A, which is a flow chart illustrating the code card reading method provided in one embodiment of the present invention and includes the following steps:
Step 301: inserting the code card 10 into the slot 103 of the electrochemical strip reading apparatus 100.
Step 302: when the metal pin area 11 of the code card 10 is connected with the slot 103 of the electrochemical strip reading apparatus 100 to recognize the code card 10, a plurality of the metal pins 1101, 1102 of the code card 10 are used as data transmitting pins.
Step 303: activating the electrochemical strip reading apparatus 100 (the activating method can be automatic or manual, and automatic activating is preferably used in the present embodiment).
Step 304: reading the identifying information and the standard resistor reference value stored in the memory chip 12 of the code card 10, and the identifying information includes the type, the serial number, the calculation formula, the expired date and the reagent correction data of the electrochemical strip 20.
Step 305: on the other hand, the pins of the slot 103 is connected with the metal pin area 11 of the code card 10 to detect the standard resistor 13 on the code card 10 to read the standard resistor signal and compare with the standard resistor reference value to obtain a calibration deviation value.
Step 306: the calibration deviation value is stored in the database module 1051 of the electrochemical strip reading apparatus 100 to be a compensation value for the following detection of the electrochemical strip 20 to automatically compensate the detecting error.
Step 307: finishing the memory correction procedure.
Step 308: optionally restoring the detecting result, the setting warning data, and the operating information data of the electrochemical strip reading apparatus 100 into the memory chip 12 of the code card 10.
Step 309: finishing the data storing procedure.

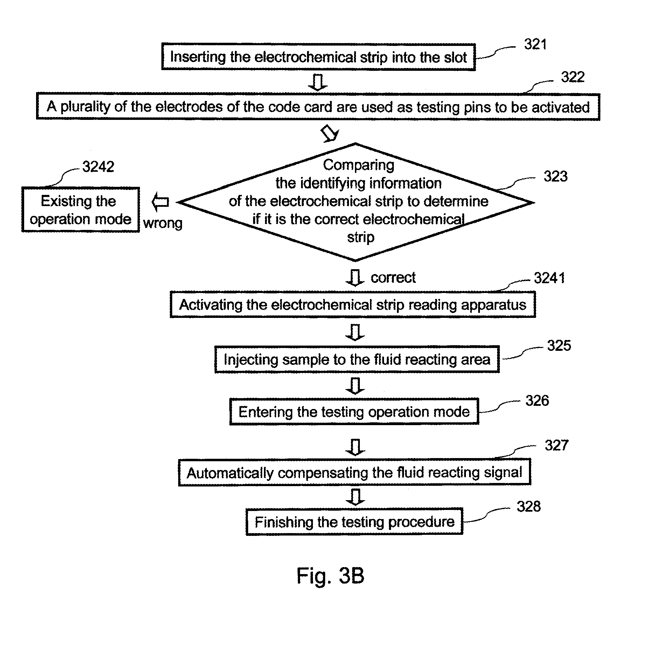

Please refer to FIG. 3B, which is a flow chart illustrating an electrochemical strip 20 reading method provided in one embodiment of the present invention and includes the following steps:
Step 321: inserting the electrochemical strip 20 into the slot 103 of the electrochemical strip reading apparatus 100.
Step 322: when the electrode area 21 of the electrochemical strip 20 is connected with the slot 103 of the electrochemical strip reading apparatus 100 to recognize the he electrochemical strip 20, a plurality of the electrodes 2101, 2102 of the electrochemical strip 20 are used as testing pins to be activated.
Step 323: comparing the identifying information of the electrochemical strip 20 to determine if it is the correct electrochemical strip 20.
Step 3241: when it is the correct electrochemical strip 20, the electrochemical strip reading apparatus 100 is activated (skip to step 325).
Step 3242: if it is the wrong electrochemical strip 20, the electrochemical strip 20 is removed and exits the operation mode.
Step 325: injecting the sample (blood or body fluid) to the fluid reacting area 22 of the electrochemical strip 20.
Step 326: the electrochemical strip reading apparatus 100 is in testing operation mode to read the fluid reacting signal generated from the fluid reacting area 22 of the electrochemical strip 20.
Step 327: automatically compensating the fluid reacting signal by the calibration deviation value calculated in Step 305 and Step 306 of the code card reading method.
Step 328: finishing the testing procedure.

Although some particular embodiments of the invention have been described in detail for purposes of illustration, it will be understood by one of ordinary skill in the art that numerous variations will be possible to the disclosed embodiments without going outside the scope of the invention as disclosed in the claims.

## Claims

1. An electrochemical strip reading apparatus (100) applied in detecting an electrochemical strip (20), comprising:
an individual code card (10) including a metal pin area (11), a memory chip (12) and a standard resistor (13), wherein the memory chip (12) stores an identifying information of the electrochemical strip (20) and a standard resistor reference value;
a housing (101);
a monitor (102) disposed on the housing (101);
a slot (103) being disposed on the housing (101) and including a plurality of pins (1031, 1032) for insertion of the code card (10) and the electrochemical strip (20);
a current detecting and converting circuit (104) disposed within the housing (101) to read a standard resistor signal of the standard resistor (13) of the code card (10) and read a fluid reacting signal of a fluid reacting area (22) of the electrochemical strip (20) via the slot (103); and
a microprocessor (105) disposed within the housing (10) including:
a database module (1051) storing the identifying information and other identifying information of other electrochemical strips, the stand resistor reference value, the standard resistor signal, and the fluid reacting signal; and
a calculating module (1052) reading the identifying information of the electrochemical strip (20) from the database module (1051), comparing the fluid reacting signal and the identifying information, and calculating out a detecting result ;
wherein the code card (10) is connected to the slot (103) via the metal pin area (11) and the electrochemical strip reading apparatus (100) recognizes the code card (10) and reads the identifying information and the standard resistor reference value stored in the memory chip (12) and reads the standard resistor signal of the standard resistor (13).

2. The electrochemical strip reading apparatus (100) of claim 1, wherein the electrochemical strip (20) is connected to the slot (103) and the electrochemical strip reading apparatus (100) uses a calibration deviation value generated by comparing the standard resistor signal with the standard resistor reference value to correct a detecting error generated from reading the electrochemical strip (20).

3. The electrochemical strip reading apparatus (100) of claim 1, wherein the identifying information stored in the memory chip (12) of the code card (10) includes a type, a serial number, a calculation formula, an expired date and a reagent correction data of the electrochemical strip (20).

4. The electrochemical strip reading apparatus (100) of claim 1, wherein a plurality of the metal pins (1101, 1102) formed in the metal pin area (11) of the code card (10) are connected to the pins (1031, 1032) of the slot (103), the electrochemical strip reading apparatus (100) is activated to read the identifying information and the standard resistor reference value stored in the code card (10), and the identifying information and the standard resistor reference value are stored in the database module (1051) of the electrochemical strip reading apparatus (100).

5. The electrochemical strip reading apparatus (100) of claim 4, wherein the code card (10) is connected to the electrochemical strip reading apparatus (100), the electrochemical strip readying apparatus (100) reads the standard resistor signal of the standard resistor (13) of the code card (10) and the standard resistor signal is stored and calculated by the microprocessor (105).

6. The electrochemical strip reading apparatus (100) of claim 1, wherein the electrochemical strip (20) includes an electrical area (21) formed with a plurality of electrodes (2101, 2102) for a respective connection with the pins (1031, 1032) of the slot (103), and the respective connection enables the electrochemical strip reading apparatus (100) to recognize the electrochemical strip (20).

7. The electrochemical strip reading apparatus (100) of claim 6, wherein the electrochemical strip (20) is recognized, the electrochemical strip reading apparatus (100) is activated, and the current detecting and converting circuit (104) reads the fluid reacting signal of the fluid reacting area (22) of the electrochemical strip (20).

8. The electrochemical strip reading apparatus (100) of claim 7, wherein the fluid reacting signal is compensated by the calibration deviation value and calculated by the calculating module (1052) to obtain the detecting result.

9. The electrochemical strip reading apparatus (100) of claim 1, wherein the memory chip (12) of the code card (10) further accesses a plurality of the detecting results stored in the database module (1051), and serves as a bi-directional transmitting interface between the electrochemical strip reading apparatus (100) and the code card (10).

10. An electrochemical strip reading method, comprising steps of:
providing an electrochemical strip reading apparatus (100) disposed with a slot (103);
reading a code card including steps of:
providing a code card (10) having a metal pin area (11), a memory chip (12) and a standard resistor (13);
inserting the code card (10) into the slot (103) and serving a plurality of metal pins (1101, 1102) of the metal pin area (11) of the code card (10) as data transmitting pins and activating the electrochemical strip reading apparatus (100);
reading an identifying information and a standard resistor reference value stored in the memory chip (12) with the identifying information including a type, a serial number, a calculation formula, an expired date and a reagent correction data of the electrochemical strip (20), and storing the identifying information and the standard resistor reference value in a database module (1051) of the electrochemical strip reading apparatus (100);
detecting a standard resistor signal of the standard resistor (13), comparing the standard resistor signal with the standard resistor reference value to generate a calibration deviation value, and storing the calibration deviation value to enable a compensation of a detecting result of the electrochemical strip (20); and
restoring the detecting results, a setting warning information, and an operating information of the electrochemical strip reading apparatus (100) to the memory chip (12) and completing a reading and writing procedure of the code card (10); and
reading an electrochemical strip including steps of :
providing an electrochemical strip (20) disposed with an electrical area (21) and a fluid reacting area (22);
inserting the electrochemical strip (20) into the slot (103) and activating a plurality of electrodes (2101, 2102) of the electrical area (21);
injecting a sample in the fluid reacting area (22) of the electrochemical strip (20) for detecting;
reading a fluid reacting signal generated by the fluid reacting area (22);
compensating the fluid reacting signal;
calculating and outputting a detecting result according to the compensated fluid reacting signal; and
completing the detecting procedure of the electrochemical strip (20).
